# EUROPÄISCHE PATENTSCHRIFT

(11) **EP 0 695 303 B1**
(45) Veröffentlichungstag und Bekanntmachung des Hinweises auf die Patenterteilung: **28.11.2001**
(21) Anmeldenummer: 94915070.0
(22) Anmeldetag: 20.04.1994
(51) Int. Cl.: C07D 471/04, A61K 31/44, A61P 31/04

(54) **PYRIDINIUMSALZE UND IHRE VERWENDUNG ZUR BEKÄMPFUNG VON HELICOBACTER-BAKTERIEN**
PYRIDINIUM SALTS AND THEIR USE FOR CONTROLLING HELICOBACTER BACTERIA
SELS DE PYRIDINIUM ET LEUR UTILISATION POUR COMBATTRE DES BACTERIES HELICOBACTER

(30) Priorität: 22.04.1993 CH 123293
(43) Veröffentlichungstag der Anmeldung: 07.02.1996
(73) Patentinhaber: Byk Gulden Lomberg Chemische Fabrik GmbH, 78467 Konstanz (DE)
(72) Erfinder: SENN-BILFINGER, Jörg, D-78464 Konstanz (DE); GRUNDLER, Gerhard, D-78464 Konstanz (DE); RAINER, Georg, D-78464 Konstanz (DE); OPFERKUCH, Wolfgang, D-44801 Bochum (DE)
(86) Internationale Anmeldenummer: EP9401218
(87) Internationale Veröffentlichungsnummer: WO9424130

(56) Entgegenhaltungen:
- EP-A- 0 033 094
- BIOCHEMICAL PHARMACOLOGY Bd. 37, Nr. 11 , 1988 , GB Seiten 2231 - 2236 D. J. KEELING ET AL 'SCH 28080 is a lumenally acting, potassium site inhibitor of the gastric proton-potassium ATPase'

## Beschreibung

### Anwendungsgebiet der Erfindung

Die Erfindung betrifft neue Pyridiniumsalze und ihre Verwendung zur Herstellung von Arzneimitteln, die zur Behandlung von Erkrankungen des Magens und/oder Darms eingesetzt werden sollen, die durch Helicobacter-Bakterien hervorgerufen werden.

### Stand der Technik

In einer Vielzahl von europäischen Patentanmeldungen werden unterschiedlich substituierte Imidazo[1,2-a]pyridine beschrieben (so z.B. in EP-A-0 033 094, EP-A-0 068 378, EP-A-0 120 589, EP-A-0 204 285, EP-A-0 228 006, EP-A-0 266 890, EP-A-0 268 989 und EP-A-0 308 917), die sich zur Verhütung und Behandlung von Ulcuserkrankungen des Magens eignen sollen. Den in den genannten europäischen Patentanmeldungen offenbarten lmidazo[1,2-a]pyridinen ist gemeinsam, daß sie in 1-Position nicht substituiert sind. In Biochem. Pharm. 37 (1988), 2231-2236 wird ein in 1-Position methyliertes Imidazo[1,2-a]pyridin beschrieben, das die Aktivität der (K⁺/H⁺)-ATPase des Magens herabsetzen soll.

### Beschreibung der Erfindung

Überraschenderweise wurde nun gefunden, daß die nachfolgend beschriebenen Pyridiniumsalze, die sich von den Imidazo[1,2-a]pyridinen des Standes der Technik insbesondere durch den Substituenten in 1-Position unterscheiden, gegen Helicobacter-Bakterien wirksam sind.

Gegenstand der Erfindung sind somit die Verbindungen der Formel I (siehe beiliegendes Formelblatt), worin
- R1: CₙH₂ₙ-A darstellt, wobei
- A: Wasserstoff (H), Naphthyl, Phenyl oder durch einen oder zwei gleiche oder verschiedene Substituenten aus der Gruppe Brom, Chlor, Fluor, 1-4C-Alkyl, Phenyl, 1-4C-Alkoxy, Cyan, 1-4C-Alkoxycarbonyl, Trifluormethyl und Trifluormethoxy substituiertes Phenyl bedeutet und
- n: die Zahl 1, 2, 3 oder 4 bedeutet,
- R2: 1-4C-Alkyl bedeutet,
- R3: 1-4C-Alkyl, 3-4C-Alkinyl, Hydroxy-1-4C-alkyl oder Cyanmethyl bedeutet,
- m: die Zahl 1 oder 2 bedeutet und
- X^{θ}: ein geeignetes Anion darstellt,
wobei R1 nicht Methyl bedeutet, wenn R2 Methyl, R3 Cyanmethyl und m die Zahl 1 bedeuten.

Mit A = Wasserstoff (H) und n = 1, 2, 3 oder 4 bedeutet CₙH₂ₙ-A 1-4C-Alkyl.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkoxy steht für einen Rest, der neben dem Sauerstoffatom einen der vorstehend genannten 1-4C-Alkylreste enthält. Bevorzugt ist der Methoxyrest.

1-4C-Alkoxycarbonyl steht für einen Rest, der neben der Carbonylgruppe einen der vorstehend genannten 1-4C-Alkoxyreste enthält. Bevorzugt sind der Methoxycarbonyl und der Ethoxycarbonylrest.

3-4C-Alkinyl steht für einen geradkettigen oder verzweigten Alkinylrest mit 3 bis 4 Kohlenstoffatomen. Als bevorzugter Alkinylrest sei der Propinylrest genannt.

Als geeignete Anionen X^{θ} kommen prinzipiell alle Anionen infrage, besonders aber solche Anionen, die in den für die Herstellung der Verbindungen I benötigten Alkylierungsmitteln R1-X ohnehin schon vorhanden sind, oder solche Anionen, die bei Wirkstoffen in Arzneimitteln üblicherweise Verwendung finden. Beispielsweise seien das Chlorid-, das Bromid-, das Jodid- und das Methylsulfation genannt.

Hervorzuhebende Verbindungen sind solche der Formel I,
wobei
- A: Wasserstoff (H), Naphthyl, Phenyl oder durch einen oder zwei gleiche oder verschiedene Substituenten aus der Gruppe Brom, Chlor, Fluor, 1-4C-Alkyl, Phenyl, 1-4C-Alkoxycarbonyl und Trifluormethyl substituiertes Phenyl bedeutet und
- n: die Zahl 1 oder 2 bedeutet,
- R3: 1-4C-Alkyl, 3-4C-Alkinyl oder Cyanmethyl bedeutet und
- m: die Zahl 1 bedeutet.

Besonders hervorzuhebende Verbindungen sind solche der Formel I, wobei
- A: Phenyl oder durch einen oder zwei gleiche oder verschiedene Substituenten aus der Gruppe Chlor, Fluor, Methyl, Methoxy, Cyan, Methoxycarbonyl, Trifluormethyl und Trifluormethoxy substituiertes Phenyl bedeutet und
- n: die Zahl 1 oder 2 bedeutet,
- R2: Methyl oder Ethyl bedeutet,
- R3: Methyl, Propinyl, Hydroxymethyl oder Cyanmethyl bedeutet und
- m: die Zahl 1 bedeutet.

Weiterer Gegenstand der Erfindung ist ein Verfahren zur Herstellung der erfindungsgemäßen Verbindungen und ihrer Salze. Das Verfahren ist dadurch gekennzeichnet, daß man Verbindungen der Formel II (siehe Formelblatt), worin R2, R3 und m die oben angegebenen Bedeutungen haben, mit Verbindungen der Formel III

R1 - X (III)

umsetzt, und daß man gewünschtenfalls anschließend erhaltene Verbindungen I in ihre Salze überführt, oder daß man gewünschtenfalls anschließend aus erhaltenen Salzen der Verbindungen I die Verbindungen I freisetzt.

Die Umsetzung der Verbindungen II mit den Verbindungen III erfolgt in einer dem Fachmann an sich vertrauten Weise in geeigneten, inerten Lösungsmitteln. Die nachfolgenden Beispiele dienen der näheren Erläuterung des erfindungsgemäßen Verfahrens. Die Abkürzung h steht für Stunde(n), RT für Raumtemperatur, Schmp. für Schmelzpunkt. Die in den Beispielen genannten Verbindungen und Salze dieser Verbindungen sind bevorzugter Gegenstand der Erfindung.

### Beispiele

### 1. 1-Benzyl-8-benzyloxy-2,3-dimethyl-imidazo[1,2-a]pyridinium Bromid

Eine 50°C warme Lösung von 2 g 8-Benzyloxy-2,3-dimethyl-imidazo[1,2-a]pyridin, gelöst in 40 ml wasserfreiem Aceton, wird mit 2,7 g Benzylbromid versetzt und anschließend 16 h unter Rückfluß erhitzt. Der hierbei anfallende Niederschlag wird abfiltriert und mit kaltem Aceton gewaschen. Es werden 3,5 g der Titelverbindung vom Schmp. 190-192°C erhalten.

### 2. 1-Benzyl-8-benzyloxy-3-cyanomethyl-2-methyl-imidazo[1,2-a]pyridinium Bromid

Die Titelverbindung von Schmp. 188-190°C wird analog zu Beispiel 1 durch Umsetzung von 8-Benzyloxy-3-cyanomethyl-2-methyl-imidazo[1,2-a]pyridin mit Benzylbromid erhalten.

### 3. 8-Benzyloxy-1-(4-fluorbenzyl)-2,3-dimethyl-imidazo[1,2-a]pyridinium Bromid

Die Titelverbindung vom Schmp. 188-191°C wird durch Umsetzung von 8-Benzyloxy-2,3-dimethyl-imidazo[1,2-a]pyridin mit 4-Fluorbenzylbromid analog zu Beispiel 1 erhalten.

### 4. 8-Benzyloxy-1-(4-methoxycarbonylbenzyl)-2,3-dimethyl-imidazo[1,2-a]pyridinium Bromid

Die Titelverbindung vom Schmelzbereich 177-188°C wird durch Umsetzung von 8-Benzyloxy-2,3-dimethyl-imidazo[1,2-a]pyridin mit 4-Brommethylbenzoesäuremethylester analog zu Beispiel 1 erhalten.

### 5. 8-Benzyloxy-2,3-dimethyl-1-phenethyl-imidazo[1,2-a]pyridinium Bromid

Die Titelverbindung vom Schmp. 173-176°C wird durch Umsetzung 8-Benzyloxy-2,3-dimethyl-imidazo[1,2-a]pyridin mit 2-Phenethylbromid analog zu Beispiel 1 erhalten.

### 6. 8-Benzyloxy-3-cyanomethyl-2-methyl-1-phenethyl-imidazo[1,2-a]pyridinium Bromid

Die Titelverbindung vom Schmp. 202-205°C wird durch Umsetzung von 8-Benzyloxy-3-cyanomethyl-2-methyl-imidazo[1,2-a]pyridin mit 2-Phenethylbromid analog zu Beispiel 1 erhalten.

### 7. 8-Benzyloxy-2-methyl-1-phenethyl-3-(2-propinyl)-imidazo[1,2-a]pyridinium Bromid

Die Titelverbindung vom Schmelzbereich 142-150°C wird durch Umsetzung von 8-Benzyloxy-2-methyl-3-(2-propinyl)-imidazo[1,2-a]pyridin mit 2-Phenethylbromid analog zu Beispiel 1 erhalten.

### 8. 1-Benzyl-8-benzyloxy-2-methyl-3-(2-propinyl)-imidazo[1,2-a]pyridinium Bromid

Die Titelverbindung vom Schmp. 190-192°C wird durch Umsetzung von 8-Benzyloxy-2-methyl-3-(2-propinyl)-imidazo[1,2-a]pyridin mit Benzylbromid analog zu Beispiel 1 erhalten.

### 9. 8-Benzyloxy-2,3-dimethyl-1-(4-methylbenzyl)-imidazo[1,2-a]pyridinium Bromid

Die Titelverbindung vom Schmp. 212-215°C wird durch Umsetzung von 8-Benzyloxy-2,3-dimethyl-imidazo[1,2-a]pyridin mit 4-Methylbenzylbromid analog zu Beispiel 1 erhalten.

### 10. 8-Benzyloxy-1-(3,5-difluorbenzyl)-2,3-dimethyl-imidazo[1,2-a]pyridinium Bromid

Die Titelverbindung vom Schmp. 195-196°C wird durch Umsetzung von 8-Benzyloxy-2,3-dimethyl-imidazo[1,2-a]pyridin mit 3,5-Difluorbenzylbromid analog zu Beispiel 1 erhalten.

### 11. 8-Benzyloxy-1-(3-trifluormethylbenzyl)-2,3-dimethyl-imidazo[1,2-a]pyridinium Bromid

Die Titelverbindung vom Schmp. 187-189°C wird durch Umsetzung von 8-Benzyloxy-2,3-dimethyl-imidazo[1,2-a]pyridin mit 3-Trifluormethylbenzylbromid analog zu Beispiel 1 erhalten.

### 12. 8-Benzyloxy-2,3-dimethyl-1-(2-naphthylmethyl)-imidazo[1,2-a]pyridinium Bromid

Die Titelverbindung vom Schmp. 195-197°C wird durch Umsetzung von 8-Benzyloxy-2,3-dimethyl-imidazo[1,2-a]pyridin mit 2-Brommethylnaphthalin analog zu Beispiel 1 erhalten.

### 13. 8-Benzyloxy-1-(3-cyanobenzyl)-2,3-dimethyl-imidazo[1,2-a]pyridinium Bromid

Die Titelverbindung vom Schmp. 193-195°C wird durch Umsetzung von 8-Benzyloxy-2,3-dimethyl-imidazo[1,2-a]pyridin mit 3-Brommethylbenzonitril analog zu Beispiel 1 erhalten.

### 14. 8-Benzyloxy-1-(4-chlorbenzyl)-2,3-dimethyl-imidazo[1,2-a]pyridinium Bromid

Die Titelverbindung vom Schmp. 215-217°C wird durch Umsetzung von 8-Benzyloxy-2,3-dimethyl-imidazo[1,2-a]pyridin mit 4-Chlorbenzylbromid analog zu Beispiel 1 erhalten.

### 15. 8-Benzyloxy-1-(3,5-dimethylbenzyl)-2,3-dimethyl-imidazo[1,2-a]pyridinium Bromid

Die Titelverbindung vom Schmp. 205-207°C wird durch Umsetzung von 8-Benzyloxy-2,3-dimethyl-imidazo[1,2-a]pyridin mit 3,5-Dimethylbenzylbromid analog zu Beispiel 1 erhalten.

### 16. 8-Benzyloxy-1-(3,4-dichlorbenzyl)-2,3-dimethyl-imidazo[1,2-a]pyridinium Bromid

Die Titelverbindung vom Schmp. 199-201°C wird durch Umsetzung von 8-Benzyloxy-2,3-dimethyl-imidazo[1,2-a]pyridin mit 3,4-Dichlorbenzylbromid analog zu Beispiel 1 erhalten.

### 17. 8-Benzyloxy-1-(3,5-bis(trifluormethyl)benzyl)-2,3-dimethyl-imidazo[1,2-a]pyridinium Bromid

Die Titelverbindung vom Schmp. 183-186°C wird durch Umsetzung von 8-Benzyloxy-2,3-dimethyl-imidazo[1,2-a]pyridin mit 3,5-Bis(trifluormethyl)-benzylbromid analog zu Beispiel 1 erhalten.

### 18. 8-Benzyloxy-1-(4-biphenylmethyl)-2,3-dimethyl-imidazo[1,2-a]pyridinium Bromid

Die Titelverbindung vom Schmp. 145-150°C wird durch Umsetzung von 8-Benzyloxy-2,3-dimethyl-imidazo[1,2-a]pyridin mit 4-Brommethylbiphenyl analog zu Beispiel 1 erhalten.

### 19. 8-Benzyloxy-3-cyanomethyl-1,2-dimethyl-imidazo[1,2-a]pyridinium Jodid

Die Titelverbindung vom Schmp. 185-186°C wird durch Umsetzung von 8-Benzyloxy-3-cyanomethyl-2-methyl-imidazo[1,2-a]pyridin mit Methyljodid analog zu Beispiel 1 erhalten.

### 20. 8-Benzyloxy-1,2,3-trimethyl-imidazo[1,2-a]pyridinium Jodid

Die Titelverbindung vom Schmp. 218-219°C wird durch Umsetzung von 8-Benzyloxy-2,3-dimethyl-imidazo[1,2-a]pyridin mit Methyljodid analog zu Beispiel 1 erhalten.

### 21. 8-Benzyloxy-3-cyanomethyl-1,2,-dimethyl-imidazo[1,2-a]pyridinium Methosulfat

Eine Lösung von 100 mg 8-Benzyloxy-3-cyanomethyl-2-methyl-imidazo[1,2-a]pyridin in 3 ml trockenem Aceton wird mit 45 mg Dimethylsulfat ersetzt und bei RT 16 h gerührt. Der hierbei gebildete Niederschlag wird abfiltriert, mit wenig Diethylether gewaschen und getrocknet. Es werden 90 mg der Titelverbindung vom Schmp. 185-187°C erhalten.

### 22. 8-Benzyloxy-1-(4-trifluormethylbenzyl)-2,3-dimethyl-imidazo[1,2-a]pyridinium Bromid

Die Titelverbindung vom Schmp. 207-209°C wird durch Umsetzung von 8-Benzyloxy-2,3-dimethyl-imidazo[1,2-a]pyridin mit 4-Trifluormethylbenzylbromid analog zu Beispiel 1 erhalten.

### 23. 8-Benzyloxy-1-(3,4-difluorbenzyl)-2,3-dimethyl-imidazo[1,2-a]pyridinium Bromid

Die Titelverbindung vom Schmp. 191-193°C wird durch Umsetzung von 8-Benzyloxy-2,3-dimethyl-imidazo[1,2-a]pyridin mit 3,4-Difluorbenzylbromid analog zu Beispiel 1 erhalten.

### 24. 8-Benzyloxy-1-(3-chlorbenzyl)-2,3-dimethyl-imidazo[1,2-a]pyridinium Bromid

Die Titelverbindung vom Schmp. 183-185°C wird analog Beispiel 23 durch Umsetzung mit 3-Chlorbenzylbromid erhalten.

### 25. 8-Benzyloxy-1-(4-trifluormethoxybenzyl)-2,3-dimethyl-imidazo[1,2-a]pyridinium Bromid

Die Titelverbindung vom Schmp. 148-151°C wird analog zu Beispiel 23 durch Umsetzung mit 4-Trifluormethoxybenzylbromid erhalten.

### 26. 8-Benzyloxy-1-(3-trifluormethoxybenzyl)-2,3-dimethyl-imidazo[1,2-a]pyridinium Bromid

Die Titelverbindung vom Schmp. 137-140°C wird analog zu Beispiel 23 durch Umsetzung mit 3-Trifluormethoxybenzylbromid erhalten.

### 27. 8-Benzyloxy-1-(3-methylbenzyl)-2,3-dimethyl-imidazo[1,2-a]pyridinium Bromid

Die Titelverbindung vom Schmp. 180-182°C wird analog zu Beispiel 23 durch Umsetzung mit 3-Methylbenzylbromid erhalten.

### 28. 8-Benzyloxy-1-(4-t-butylbenzyl)-2,3-dimethyl-imidazo[1,2-a]pyridinium Bromid

Die Titelverbindung vom Schmp. 200-202°C wird analog zu Beispiel 23 durch Umsetzung mit 4-t-Butylbenzylbromid erhalten.

### 29. 8-Benzyloxy-2-methyl-1-(3,5-dimethylbenzyl)-3-(2-propinyl)-imidazo-[1,2-a]pyridinium Bromid

Die Titelverbindung vom Schmp. 200-202°C wird durch Umsetzung von 8-Benzyloxy-2-methyl-3-(2-propinyl)-imidazo[1,2-a]pyridin mit 3,5-Dimethylbenzylbromid analog zu Beispiel 1 erhalten.

### 30. 8-Benzyloxy-1-(4-cyanobenzyl)-2,3-dimethyl-imidazo[1,2-a]pyridinium Bromid

Die Titelverbindung vom Schmp. 143-147°C wird durch Umsetzung mit 4-Cyanobenzylbromid analog zu Beispiel 23 erhalten.

### 31. 8-Benzyloxy-2,3-dimethyl-1-(2,4-dimethylbenzyl)-imidazo[1,2-a]pyridinium Chlorid

Die Titelverbindung vom Schmp. 192-195°C wird analog zu Beispiel 23 durch Umsetzung mit 2,4-Dimethylbenzylchlorid erhalten.

### 32. 8-Benzyloxy-2,3-dimethyl-1-(2,5-dimethylbenzyl)-imidazo[1,2-a]pyridinium Chlorid

Die Titelverbindung vom Schmp. 197-200°C wird analog zu Beispiel 23 durch Umsetzung mit 2,5-Dimethylbenzylchlorid erhalten.

### 33. 8-Benzyloxy-2,3-dimethyl-1-(3,5-dimethylbenzyl)-imidazo[1,2-a]pyridinium Chlorid

Die Titelverbindung vom Schmp. 168-173°C wird analog zu Beispiel 23 durch Umsetzung mit 3,5-Dimethylbenzylchlorid erhalten.

### 34. 8-Benzyloxy-1-ethyl-2,3-dimethyl-imidazo[1,2-a]pyridinium Bromid

Die Titelverbindung vom Schmp. 193-195°C wird analog zu Beispiel 23 durch Umsetzung mit Ethylbromid erhalten.

### 35. 8-Benzyloxy-1-n-propyl-2,3-dimethyl-imidazo[1,2-a]pyridinium Bromid

Die Titelverbindung vom Schmp. 206-209°C wird analog zu Beispiel 23 durch Umsetzung mit 1-Brompropan erhalten.

### 36. 8-Benzyloxy-1-n-butyl-2,3-dimethyl-imidazo[1,2-a]pyridinium Bromid

Die Titelverbindung vom Schmp. 172-174°C wird analog zu Beispiel 23 durch Umsetzung mit 1-Brombutan erhalten.

### 37. 2,3-Dimethyl-1-(3,5-dimethylbenzyl)-8-(2-phenethoxy)-imidazo[1,2-a]pyridinium Bromid

Die Titelverbindung vom Schmp. 198-200°C wird analog zu Beispiel 1 durch Umsetzung von 23-Dimethyl-8-(2-phenethoxy)-imidazo[1,2-a]pyridin mit 3,5-Dimethylbenzylbromid erhalten.

### 38. 8-Benzyloxy-2-ethyl-3-methyl-1-(3,5-dimethylbenzyl)-imidazo[1,2-a]pyridinium Bromid

Die Titelverbindung vom Schmp. 214-216°C wird analog zu Beispiel 1 durch Umsetzung von 8-Benzyloxy-2-ethyl-3-methyl-imidazo[1,2-a]pyridin mit 3,5-Dimethylbenzylbromid erhalten.

### 39. 8-Benzyloxy-2-ethyl -3-hydroxymethyl-1-(3,5-dimethylbenzyl)-imidazo[1,2-a]pyridinium Bromid

Die Titelverbindung vom Schmp. 208-210°C wird analog zu Beispiel 1 durch Umsetzung von 8-Benzyloxy-2-ethyl-3-hydroxymethyl-imidazo[1,2-a]pyridin mit 3,5-Dlmethylbenzylbromid erhalten.

### 40. 8-Benzyloxy-1-(3-methoxycarbonylbenzyl)-2,3-dimethyl-imidazo[1,2-a]pyridinium Bromid

Die Titelverbindung vom Schmp. 150-154°C wird analog zu Beispiel 23 durch Umsetzung mit 3-Methoxycarbonylbenzylbromid erhalten.

### 41. 8-Benzyloxy-1-(3-methoxybenzyl)-2,3-dimethyl-imidazo[1,2-a]pyridinium Chlorid

Die Titelverbindung vom Schmp. 144-148°C wird analog zu Beispiel 23 durch Umsetzung mit 3-Methoxybenzylchlorid erhalten.

### 42. 8-Benzyloxy-1-(3,4-dimethylbenzyl)-2,3-dimethyl-imidazo[1,2-a]pyridinium Chlorid

Die Titelverbindung vom Schmp. 196-201°C wird analog zu Beispiel 23 durch Umsetzung mit 3,4-Dimethylbenzylchlorid erhalten.

### Gewerbliche Anwendbarkeit

Die Verbindungen der Formel I, worin R1, A, n, R2, R3, m und X^{θ} die oben angegebenen Bedeutungen haben, und ihre Salze besitzen wertvolle pharmakologische Eigenschaften, die sie gewerblich verwertbar machen. Sie weisen insbesondere eine antiulcerogene Wirksamkeit und eine ausgeprägte Wirksamkeit gegen Helicobacter-Bakterien auf. Darüberhinaus zeichnen sich die erfindungsgemäßen Verbindungen durch eine hohe Wirkungsselektivität, das Fehlen wesentlicher Nebenwirkungen und eine große therapeutische Breite aus.

Die ausgezeichnete Wirksamkeit von Verbindungen der Formel I und ihren Salzen gegen Helicobacter-Bakterien gestattet ihren Einsatz in der Humanmedizin als Wirkstoffe für die Behandlung von Ulcuserkrankungen sowie von Krankheiten, die auf Helicobacter-Bakterien beruhen.

Die Verbindungen der Erfindung eignen nich daher zur Behandlung von Säugern, insbesondere Menschen, die an Ulcuserkrankungen sowie an Krankheiten erkrankt sind, die auf Helicobacter-Bakterien beruhen. Die Behandlung ist dadurch gekennzeichnet, daß man dem erkrankten Individuum eine therapeutisch wirksame und pharmakologisch verträgliche Menge einer oder mehrerer Verbindungen der Formel I und/oder ihrer pharmakologisch verträglichen Salze verabreicht.

Gegenstand der Erfindung sind außerdem die Verbindungen der Formel I und ihre pharmakologisch verträglichen Salze zur Anwendung bei der Behandlung von Ulcuserkrankungen sowie von Krankheiten, die auf Helicobacter-Bakterien beruhen.

Ebenso umfaßt die Erfindung die Verwendung von Verbindungen der Formel I und ihren pharmakologisch verträglichen Salzen bei der Herstellung von Arzneimitteln, die zur Bekämpfung von Ulcuserkrankungen sowie solcher Krankheiten eingesetzt werden, die auf Helicobacter-Bakterien beruhen.

Ein weiterer Gegenstand der Erfindung sind Arzneimittel zur Behandlung von Ulcuserkrankungen sowie zur Bekämpfung von Helicobacter-Bakterien, die eine oder mehrere Verbindungen der allgemeinen Formel I und/oder ihre pharmakologisch verträglichen Salze enthalten.

Von den Helicobacter-Stämmen, gegenüber denen sich die Verbindungen der Formel I als wirksam erweisen, sei insbesondere der Stamm Helicobacter pylori erwähnt.

Die Arzneimittel werden nach an sich bekannten, dem Fachmann geläufigen Verfahren hergestellt. Als Arzneimittel werden die pharmakologisch wirksamen Verbindungen der Formel I und ihre Salze (=Wirkstoffe) entweder als solche, oder vorzugsweise in Kombination mit geeigneten pharmazeutischen Hilfsstoffen z.B. in Form von Tabletten, Dragees, Kapseln, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder Permeationspromotoren und Komplexbildner (z.B. Cyclodextrine) verwendet werden.

Die Wirkstoffe können beispielsweise parenteral (z.B. intravenös) oder insbesondere oral appliziert werden.

Im allgemeinen werden in der Humanmedizin die Wirkstoffe in einer Tagesdosis von 0,5 bis 50, vorzugsweise 1 bis 30 mg/kg Körpergewicht, gegebenenfalls in Form mehrerer, vorzugsweise 2 bis 6 Einzelgaben zur Erzielung des gewünschten Ergebnisses verabreicht.

### Biologische Untersuchunqen

Die erfindungsgemäßen Verbindungen wurden bezüglich ihrer Wirksamkeit gegenüber Helicobacter pylori in Anlehnung an die von Tomoyuki Iwahi et al. (Antimicrobial Agents and Chemotherapy, 1991, 490-496) beschriebene Methodik unter Verwendung von Columbia-agar (Oxoid) und bei einer Wachstumsperiode von 4 Tagen untersucht. Für die untersuchten Verbindungen ergaben sich hierbei die in der nachfolgenden Tabelle aufgeführten MIC-Werte (die angegebenen Nummern der Verbindungen stimmen mit den Nummern der Beispiele überein).

| Verbindung Nr. | MIC-Wert (µg/ml) |
|---|---|
| 3 | ≤ 10 |
| 4 | ≤ 10 |
| 5 | ≤ 10 |
| 6 | ≤ 10 |
| 7 | ≤ 10 |
| 10 | ≤ 10 |
| 12 | ≤ 10 |
| 13 | ≤ 10 |
| 18 | ≤ 10 |

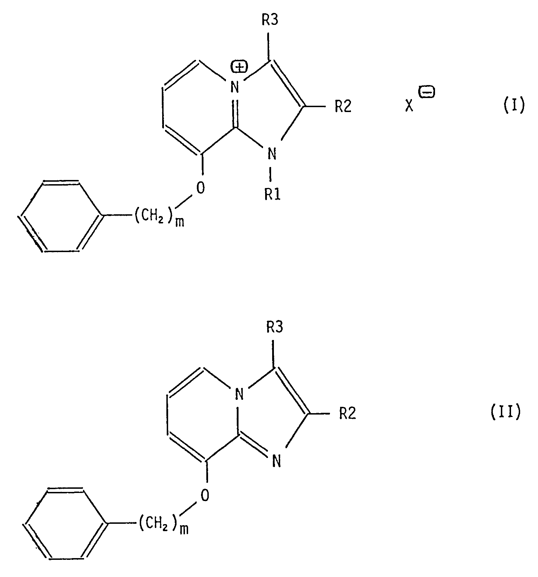

## Patentansprüche

1. Verbindungen der Formel I 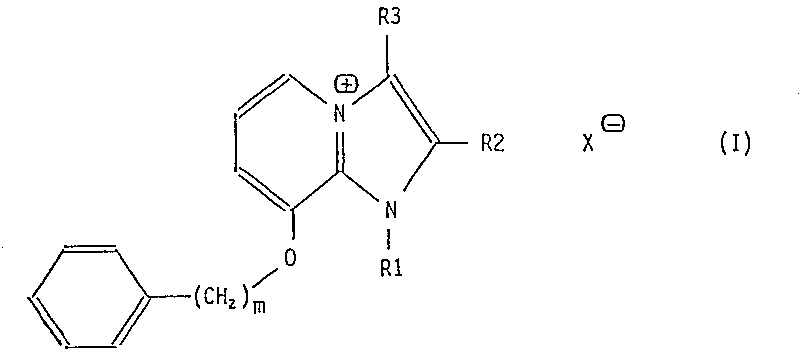 worin
R1 CₙH₂ₙ-A darstellt, wobei
A Wasserstoff (H), Naphthyl, Phenyl oder durch einen oder zwei gleiche oder verschiedene Substituenten aus der Gruppe Brom, Chlor, Fluor, 1-4C-Alkyl, Phenyl, 1-4C-Alkoxy, Cyan, 1-4C-Alkoxycarbonyl, Trifluormethyl und Trifluormethoxy substituiertes Phenyl bedeutet und
n die Zahl 1, 2, 3 oder 4 bedeutet,
R2 1-4C-Alkyl bedeutet,
R3 1-4C-Alkyl, 3-4C-Alkinyl, Hydroxy-1-4C-alkyl oder Cyanmethyl bedeutet,
m die Zahl 1 oder 2 bedeutet und
X^{θ} ein geeignetes Anion darstellt,
wobei R1 nicht Methyl bedeutet, wenn R2 Methyl, R3 Cyanmethyl und m die Zahl 1 bedeuten.

2. Verbindungen nach Anspruch 1,
wobei
A Wasserstoff (H), Naphthyl, Phenyl oder durch einen oder zwei gleiche oder verschiedene Substituenten aus der Gruppe Brom, Chlor, Fluor, 1-4C-Alkyl, Phenyl, 1-4C-Alkoxycarbonyl und Trifluormethyl substituiertes Phenyl bedeutet und
n die Zahl 1 oder 2 bedeutet,
R3 1-4C-Alkyl, 3-4C-Alkinyl oder Cyanmethyl bedeutet und
m die Zahl 1 bedeutet.

3. Verbindungen nach Anspruch 1,
wobei
A Phenyl oder durch einen oder zwei gleiche oder verschiedene Substituenten aus der Gruppe Chlor, Fluor, Methyl, Methoxy, Cyan, Methoxycarbonyl, Trifluormethyl und Trifluormethoxy substituiertes Phenyl bedeutet und
n die Zahl 1 oder 2 bedeutet,
R2 Methyl oder Ethyl bedeutet,
R3 Methyl, Propinyl, Hydroxymethyl oder Cyanmethyl bedeutet und
m die Zahl 1 bedeutet.

4. Verfahren zur Herstellung der Verbindungen der Formel I nach Anspruch 1 und ihrer Salze, **dadurch gekennzeichnet, daß** man Verbindungen der Formel II, 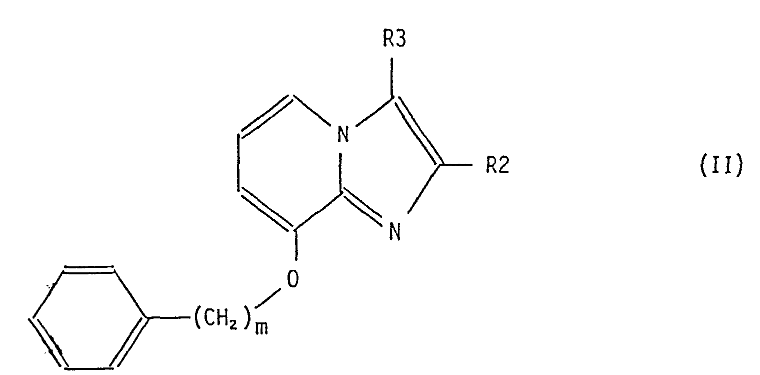 worin R2, R3 und m die in Anspruch 1 angegebenen Bedeutungen haben, mit Verbindungen der Formel III,
R1 - X (III)
worin R1 die in Anspruch 1 angegebene Bedeutung hat und X die kovalent gebundene Form des geeigneten Anions X^{θ} darstellt, umsetzt, und daß man gewünschtenfalls anschließend erhaltene Verbindungen I in ihre Salze überführt, oder daß man gewünschtenfalls anschließend aus erhaltenen Salzen der Verbindungen I die Verbindungen I freisetzt.

5. Arzneimittel enthaltend eine oder mehrere Verbindungen der Formel I nach Anspruch 1 und/oder ihre pharmakologisch verträglichen Salze.

6. Verbindungen nach Anspruch 1 zur Anwendung bei der Bekämpfung von Helicobacter-Bakterien.

7. Verwendung von Verbindungen der Formel I nach Anspruch 1 wobei R1 Methyl, R2 Methyl und R3 Cyanmethyl bedeutet, m die Zahl 1 bedeutet und X^{θ} ein geeignetes Anion darstellt, zur Herstellung von Arzneimitteln für die Bekämpfung von Helicobacter-Bakterien.

8. Anwendung gemäß Anspruch 6, **dadurch gekennzeichnet, daß** es sich bei den Helicobacter-Bakterien um Helicobacter pylori handelt.

9. Verwendung gemäß Anspruch 7, **dadurch gekennzeichnet, daß** es sich bei den Helicobacter-Bakterien um Helicobacter pylori handelt.

## Claims

1. A compound of the formula I 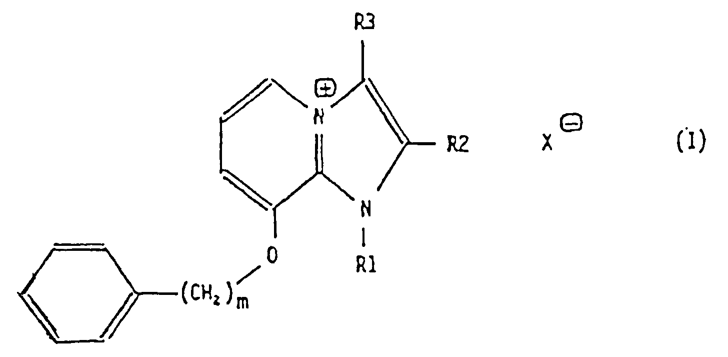 in which
R1 is CₙH₂ₙ-A, where
A is hydrogen (H), naphthyl, phenyl or phenyl which is substituted by one or two identical or different substituents from the group consisting of bromine, chlorine, fluorine, 1-4C-alkyl, phenyl, 1-4C-alkoxy, cyano, 1-4C-alkoxycarbonyl, trifluoromethyl and trifluoromethoxy, and
n is the number 1, 2, 3 or 4,
R2 is 1-4C-alkyl,
R3 1-4C-alkyl, 3-4C-alkynyl, hydroxy-1-4C-alkyl or cyanomethyl,
m is the number 1 or 2 and
X^{θ} is a suitable anion,
where R1 is not methyl if R2 is methyl, R3 is cyanomethyl and m is the number 1.

2. Compounds as claimed in claim 1,in which
A is hydrogen (H), naphthyl, phenyl or phenyl which is substituted by one or two identical or different substituents from the group consisting of bromine, chlorine, fluorine, 1-4C-alkyl, phenyl, 1-4C-alkoxycarbonyl and trifluoromethyl and
n is the number 1 or 2,
R3 is 1-4C-alkyl, 3-4C-alkynyl or cyanomethyl and
m is the number 1.

3. Compounds as claimed in claim 1,in which
A is phenyl or phenyl which is substituted by one or two identical or different substituents from the group consisting of chlorine, fluorine, methyl, methoxy, cyano, methoxycarbonyl, trifluoromethyl and trifluoromethoxy, and
n is the number 1 or 2,
R2 is methyl or ethyl
R3 is methyl, propynyl, hydroxymethyl or cyanomethyl and
m is the number 1.

4. A process for the preparation of the compounds of the formula I as claimed in claim 1, and their salts, which comprises reacting compounds of the formula II 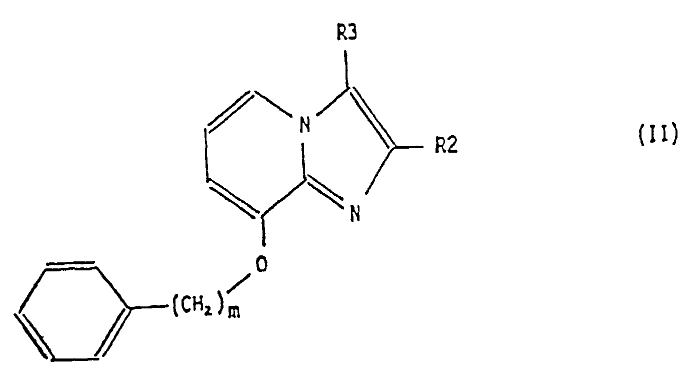 in which R2, R3 and m have the meanings indicated in claim 1, with compounds of the formula III
R1 - X (III)
in which R1 has the meaning indicated in claim 1 and X is the covalently bonded form of the suitable anion X^{θ}, and, if desired, then converting compounds I obtained into their salts, or, if desired, then liberating the compounds I from salts of the compounds I obtained.

5. A medicament comprising one or more compounds of the formula I as claimed in claim 1 and/or their pharmacologically tolerable salts.

6. Compounds as claimed in claim 1 for use in the control of Helicobacter bacteria.

7. The use of compounds of the formula I as claimed in claim 1, where R1 is methyl, R2 is methyl and R3 is cyanomethyl, m is the number 1 and X^{θ} is a suitable anion, for the production of medicaments for the control of Helicobacter bacteria.

8. The use as claimed in claim 6, wherein the Helicobacter bacteria are Helicobacter pylori.

9. The use as claimed in claim 7, wherein the Helicobacter bacteria are Helicobacter pylori.

## Revendications

1. Composés de formule (I) 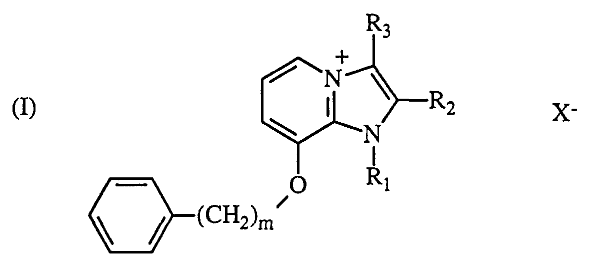 dans laquelle
R₁ représente un groupe CₙH₂ₙ-A, où A représente un atome d'hydrogène (H), un groupe naphtyle, un groupe phényle ou un groupe phényle portant un ou deux substituants, identiques ou différents, choisis dans le groupe formé par les atomes de brome, de chlore ou de fluor, les groupes alkyle en C₁₋₄, phényle, alcoxy en C₁₋₄, cyano, (alcoxy en C₁₋₄)-carbonyle, trifluorométhyle et trifluorométhoxy, et n vaut 1, 2, 3 ou 4,
R₂ représente un groupe alkyle en C₁₋₄,
R₃ représente un groupe alkyle en C₁₋₄, alcynyle en C₃₋₄, hydroxyalkyle en C₁₋₄, ou cyanométhyle,
m vaut 1 ou 2 et
X⁻ représente un anion approprié,
R₁ étant différent d'un groupe méthyle lorsque R₂ représente un groupe méthyle, R₃ un groupe cyanométhyle et m vaut 1.

2. Composés selon la revendication 1, dans lesquels
A représente un atome d'hydrogène (H), un groupe naphtyle, un groupe phényle ou un groupe phényle portant un ou deux substituants, identiques ou différents, choisis dans le groupe formé par les atomes de brome, de chlore ou de fluor, les groupes alkyle en C₁₋₄, phényle, (alcoxy en C₁₋₄)-carbonyle et trifluorométhyle, et
n vaut 1 ou 2,
R₃ représente un groupe alkyle en C₁₋₄, alcynyle en C₃₋₄ ou cyanométhyle et
m vaut 1.

3. Composés selon la revendication 1, dans lesquels A représente un groupe phényle ou un groupe phényle portant un ou deux substituants, identiques ou différents, choisis dans le groupe formé par les atomes de brome, de chlore ou de fluor, les groupes méthyle, méthoxy, cyano, méthoxycarbonyle, trifluorométhyle et trifluorométhoxy, et
n vaut 1 ou 2,
R₂ représente un groupe méthyle ou éthyle,
R₃ représente un groupe méthyle, propynyle, hydroxyméthyle ou cyanométhyle et
m vaut 1.

4. Procédé de préparation des composés de formule (I) selon la revendication 1 et de leurs sels, **caractérisé par le fait que** l'on fait réagir des composés de formule (II) 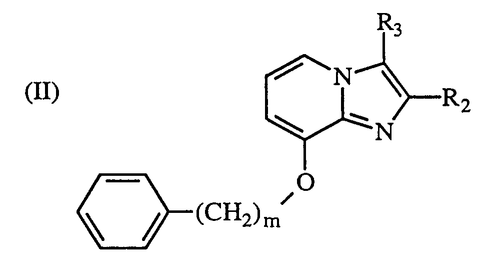 dans laquelle R₂, R₃ et m ont la signification indiquée dans la revendication 1, avec des composés de formule (III)
R₁-X (III)
dans laquelle R₁ a la signification indiquée dans la revendication 1 et X représente la forme liée par liaison covalente d'un anion X⁻ approprié, et que l'on convertit le cas échéant les composés de formule (I) obtenus, en leurs sels, ou que l'on convertit éventuellement les sels des composés de formule (I) obtenus en les composés libres correspondants.

5. Médicament contenant un ou plusieurs composés de formule (I) selon le revendication 1 et/ou les sels pharmacologiquement acceptables de ceux-ci.

6. Composés selon la revendication 1 destinés à être utilisés pour combattre des bactéries *Helicobacter.*

7. Utilisation de composés de formule (I) selon la revendication 1, dans laquelle R₁ représente un groupe méthyle, R₂ représente un groupe méthyle et R₃ un groupe cyanométhyle, m vaut 1 et X⁻ représente un anion approprié, pour la fabrication de médicaments destinés à combattre des bactéries *Helicobacter.*

8. Utilisation selon la revendication 6, **caractérisée par le fait que** les bactéries *Helicobacter* sont des *Helicobacter pylori.*

9. Utilisation selon la revendication 7, **caractérisée par le fait que** les bactéries *Helicobacter* sont des *Helicobacter pylori.*
